# EUROPÄISCHE PATENTSCHRIFT

(11) **EP 0 394 842 B1**
(45) Veröffentlichungstag und Bekanntmachung des Hinweises auf die Patenterteilung: **18.08.1993**
(21) Anmeldenummer: 90107438.5
(22) Anmeldetag: 19.04.1990
(51) Int. Cl.: B01J 23/74, B01J 23/88, C07C 29/17

(54) **Katalysator für die Hydrierung aliphatischer ungesättigter Verbindungen**
Catalyst for the hydrogenation of unsaturated aliphatic compounds
Catalyseur pour l'hydrogénation de composés aliphatiques insaturés

(30) Priorität: 27.04.1989 DE 3913835
(43) Veröffentlichungstag der Anmeldung: 31.10.1990
(73) Patentinhaber: BASF Aktiengesellschaft, 67063 Ludwigshafen (DE)
(72) Erfinder: Toussaint, Herbert, Dr., D-6710 Frankenthal (DE); Schossig, Juergen, Dr., D-6701 Fussgoenheim (DE); Graefje, Heinz, Dr., D-6700 Ludwigshafen (DE); Reiss, Wolfgang, Dr., D-6700 Ludwigshafen (DE); Spahl, Roland, Dr., D-6143 Lorsch (DE); Irgang, Mathias, Dr., D-6900 Heidelberg (DE); Himmel, Walter, Dr., D-6718 Gruenstadt (DE); Koppenhoefer, Gerhard, Dr., D-6700 Ludwigshafen (DE)

(56) Entgegenhaltungen:
- EP-A- 0 018 569
- EP-A- 0 266 727
- EP-A- 0 394 841
- FR-A- 2 305 230
- FR-A- 2 320 776
- US-A- 3 962 140

## Beschreibung

Diese Erfindung betrifft einen neuen Katalysator für die Hydrierung aliphatischer ungesättigter Verbindungen, insbesondere für die Hydrierung ungesättigter Alkohole oder Aldehyde, wie Butin-2-diol-1,4 zu Butandiol-1,4 und von 2-Ethylhexen-2-al zu 2-Ethylhexanol.

In FR-A 23 05 230 werden Trägerkatalysatoren zur Entstickung von-Abgasen beschrieben, die Oxide des Nickels und Kupfers im Atomverhältnis Ni/Cu von 1/0,05 bis 1 auf einem Träger enthalten. Dieser Träger besteht zu mindestens 50 Gew.-% aus Zirkon (ZrSiO₄) und zu höchstens 50 Gew.-% aus einem anderen hitzebeständigen Material, das z.B. Cordierit, Mullit, Magnesiumoxid, Aluminiumoxid, Silicium-Aluminium-Mischoxid, Siliciumdioxid, Zirkoniumdioxid und eine Mischung aus zwei oder mehreren dieser Materialien sein kann.

Für die Hydrierung aliphatischer ungesättigter Verbindungen sind schon zahlreiche Katalysatoren vorgeschlagen worden. Beispielsweise ist aus der US-A-3,449,445 bekannt, daß man Butin-2-diol-1,4 in guten Ausbeuten zu Butandiol-1,4 hydrieren kann, wenn man einen Katalysator verwendet, der Nickel, Kupfer und Mangan auf Siliciumdioxid enthält. Bei der großtechnischen Herstellung von Butandiol-1,4 nach diesem Verfahren treten jedoch Ablagerungen von Silicumdioxid in den Wärmeaustauschern und Rohrleitungen auf, welche sich nur durch sehr aufwendige Reinigungsoperationen entfernen lassen.

Mit dem in der DE-A-25 36 276 beschriebenen trägerfreien Katalysator, der die Oxide von Nickel, Kupfer, Molybdän und Mangan enthält, werden bei der Hydrierung acetylenisch ungesättigter Alkohole gute Ergebnisse erzielt. Allerdings müssen bei der Hydrierung von Butin-2-diol-1,4 zu Butandiol-1,4, die bei einer Verschärfung der Reaktionsbedingungen (hohe Wasserstoffdrucke und hohe Temperaturen) erhaltenen besonders günstigen Resultate mit der unerwünschten Bildung von Butanol erkauft werden. Auch sind die in Strang- oder Tablettenform hergestellten Katalysatoren im großtechnischen Einsatz nicht formbeständig, so daß ein vorzeitiger Zerfall eintritt.

Nach den Angaben in der EP-B-0 018 569 werden mit einem Hydrierkatalysator, der die Oxide der Metalle Nickel, Kupfer, Molybdän und Mangan enthält, besonders vorteilhafte Ergebnisse bei der Hydrierung von Butin-2-diol-1,4 erzielt, wenn man bei der Herstellung des Katalysators durch Fällung der Metallsalze Filtrieren, Waschen, Trocknen und Tempern vor der Ausfällung ein Aluminium- oder Eisensalz zugibt. Beim Einsatz dieses Katalysators im großtechnischen Dauerbetrieb findet jedoch eine langsame, aber kontinuierliche Auflösung des Katalysators statt.

Es bestand deshalb die Aufgabe, einen für die Hydrierung ungesättigter aliphatischer Verbindungen geeigneten Katalysator zu entwickeln, der die genannten Nachteile nicht aufweist. Der neue Katalysator sollte einerseits den für einen technischen Dauerbetrieb so wichtigen Vorteil einer hohen Beständigkeit und andererseits den Vorteil einer höheren Katalysatoraktivität aufweisen.

Es wurde nun ein für die Hydrierung aliphatischer ungesättigter Verbindungen geeigneter Katalysator gefunden, der diese Anforderungen in hohem Maße erfüllt. Der Katalysator der Nickel und Kupfer enthält, ist dadurch gekennzeichnet, daß er einen Gehalt an 20 bis 75 Gew.% Nickeloxid, 10 bis 75 Gew.% Zirkoniumdioxid und 5 bis 50 Gew.% Kupferoxid aufweist, wobei sich die Anteile jeweils auf den oxidischen nicht reduzierten Katalysator beziehen.

Der neue Katalysator eignet sich hervorragend für die Hydrierung aliphatischer ungesättigter Verbindungen. Ungesättigte aliphatische Verbindungen sind z.B. olefinisch oder acetylenische Kohlenwasserstoffe, die z.B. durch Hydroxi- oder Aldehydgruppen substituiert sein können, wie die technisch wichtigen Verbindungen Butin-2-diol-1,4, Buten-2-diol-1,4 und 2-Ethylhexen-2-al.

Der erfindungsgemäße Katalysator hat in seiner oxidischen, nicht reduzierten Form z.B. die folgende Zusammensetzung: 30 bis 70, vorzugsweise 40 bis 60, insbesondere 35 bis 55 Gew.% Nickeloxid, 10 bis 60, vorzugsweise 15 bis 50, insbesondere 25 bis 45 Gew.% Zirkoniumdioxid, 5 bis 40, vorzugsweise 10 bis 35, insbesondere 10 bis 20 Gew.% Kupferoxid. Der Katalysator kann außerdem z.B. 0,1 bis 5 Gew.% Molybdänoxid und gegebenenfalls z.B. 0 bis 10 Gew.% Manganoxid enthalten.

Ganz besonders gut ist der neue Katalysator für die Hydrierung von Butin-2-diol-1,4 zu Butandiol-1,4 geeignet. Bei dieser Hydrierung läßt sich mit dem neuen Katalysator eine erhebliche Verlängerung der Katalysatorstandzeit und eine wesentliche Steigerung des Durchsatzes erzielen.

Man stellt den neuen Katalysator z.B. dadurch her, daß man Salze der Metalle Nickel, Kupfer, Zirkonium und gegebenenfalls Mangan auf an sich übliche Weise aus wäßriger Lösung bei einer Temperatur von 30 bis 90°C und einem pH-Wert von 5 bis 9 ausfällt, die Suspension filtriert und den Filterkuchen trocknet und bei einer Temperatur von 300 bis 700°C tempert, wobei man das Molybdän als Ammoniumheptamolybdat vor dem Trocknen zugibt. Dabei nimmt man die Fällung so vor, daß man eine wäßrige Lösung von Salzen, wie den Nitraten, Sulfaten oder Acetaten, der Metalle Nickel, Kupfer, Zirkonium und gegebenenfalls Mangan mit einer wäßrigen Lösung von Alkalicarbonat mischt. Die Menge der Metallsalze wird dabei so bemessen, daß die Katalysatormasse nach dem Tempern die angegebene Zusammensetzung aufweist.

Nach einer zweckmäßigen Ausführungsform des Herstellungsverfahrens ersetzt man das wasserlösliche Zirkoniumsalz teilweise, z.B. bis zu einem Anteil von 50 Gew.%, bezogen auf das eingesetzte Zirkonium, durch festes Zirkoniumdioxid, welches man der wäßrigen Metallsalzlösung vor der Fällung zugibt oder im Reaktionsgefäß vorlegt.

Bei der Herstellung des Katalysators verfährt man im einzelnen z.B. so, daß man die wäßrige Lösung der Metallsalze gleichzeitig unter Rühren mit einer wäßrigen Alkalicarbonatlösung, vorzugsweise Natriumcarbonatlösung, vermischt, wobei die Metalle in Form eines Gemisches von Metallhydroxiden und Metallcarbonaten ausfallen. Der Metallsalzgehalt der Metallsalzlösung beträgt zweckmäßigerweise 30 bis 40 Gew.%. Die wäßrige Alkalicarbonatlösung ist z.B. 10 bis 20, vorzugsweise 15 bis 20 gew.%ig. Man fällt bei 30 bis 90, vorzugsweise bei 70 bis 90°C und einem pH-Wert von 5 bis 9, vorzugsweise 7 bis 9.

Die erhaltene Suspension wird filtriert und so lange mit Wasser gewaschen, bis keine Anionen mehr nachgewiesen werden können. Dann wird z.B. bei einer Temperatur von 120 bis 200°C im Trockenschrank oder einem Sprühtrockner getrocknet. Das Molybdän wird vorzugsweise als Ammoniumheptamolybdat dem feuchten Filterkuchen zugegeben. Der getrocknete Filterkuchen wird bei einer Temperatur von 350 bis 700°C, vorzugsweise 400 bis 600°C, getempert.

Zweckmäßigerweise wird die so erhaltene Katalysatormasse vor dem Einsatz auf an sich übliche Weise tablettiert oder extrudiert. Zum Beispiel verpreßt man die Katalysatormasse unter Verwendung eines Tablettierhilfsmittels, vorzugsweise Graphit, zu Tabletten mit den Dimensionen 6 x 3 mm. Die auf diese Weise hergestellten Tabletten werden bei einer Temperatur von 300 bis 700°C, vorzugsweise 400 bis 600°C, getempert. Die Tabletten haben ein Rüttelgewicht von 1 500 bis 1 900 g/l, eine Porosität (durch Wasseraufnahme bestimmt) von 0,2 bis 0,4 ml/g und eine Härte von 3 000 bis 4 000 N/cm². Der auf diese Weise erhältliche Katalysator wird vor seiner erfindungsgemäßen Verwendung einer reduktiven Behandlung mit Wasserstoff bei einer Temperatur von 200 bis 350°C, vorzugsweise bei 230 bis 280°C, beispielsweise über einen Zeitraum von 20 bis 40 Stunden bei Wasserstoffdrücken von 1 bis 300, vorzugsweise 100 bis 150 bar, unterworfen.

Die Hydrierung der genannten ungesättigten Verbindungen unter Verwendung des neuen Katalysators nimmt man z.B. im Temperaturbereich 40 bis 200°C und bei Drücken von 30 bis 320 bar mit einem wasserstoffhaltigen Gas vor. Die technisch besonders wichtige Hydrierung von Butin-2-diol-1,4 zu Butandiol-1,4 wird auf an sich bekannte kontinuierliche Weise in einem Reaktor durchgeführt, der den Katalysator in Form eines Festbettes enthält. Wie aus den folgenden Beispielen hervorgeht, werden hierbei erheblich längere Katalysatorstandzeiten und höhere Durchsätze als bei Verwendung der bekannten Katalysatoren erzielt.

Als ein weiterer überraschender Vorteil des erfindungsgemäßen Katalysators wurde gefunden, daß der Katalysator, der erst nach sehr langer Laufzeit desaktiviert wird, durch eine 8 bis 48 stündige Behandlung mit Wasser bei Temperaturen von 100 bis 250°C, vorzugsweise 100 bis 200°C und bei Drücken von 100 bis 320 bar nahezu vollständig reaktiviert werden kann.

### Beispiele

Die in den Beispielen genannten Prozente sind Gewichtsprozente.

### Beispiel 1

### a) Herstellung des Katalysators

Eine wäßrige Lösung aus Nickelnitrat, Kupfernitrat und Zirkonacetat, die 4,48 % NiO, 1,52 % CuO und 2,82 ZrO₂ enthielt, wurde gleichzeitig in einem Rührgefäß in einem konstanten Strom mit einer 20 %igen waßrigen Natriumcarbonatlösung bei einer Temperatur von 70°C, so gefällt, daß der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrates ca. 20 uS betrug. Dann wurde in den noch feuchten Filterkuchen so viel Ammoniumheptamolybdat eingearbeitet, daß das nachfolgend angegebene Oxidgemisch erhalten wurde. Danach wurde der Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von 4 Stunden getempert.

Der so erhaltene Katalysator hatte die Zusammensetzung: 50 % NiO, 17 % CuO, 1,5 % MoO₃ und 31,5 % ZrO₂. Das Katalysatorpulver wurde mit 3 Gew.% Graphit vermischt und zu 6 x 3-mm-Tabletten verformt. Die Tabletten hatten eine Porosität (gemessen über die Wasseraufnahme) von 0,20 ml/g und eine Härte von 3 500 N/cm².

### b) Hydrierung von Butin-2-diol-1,4

Der nach Absatz a) erhaltene Katalysator wurde in einem Hydrierreaktor bei einer Temperatur von 250°C und einem Wasserstoffdruck von 150 bar reduziert. An 8 Volumenteilen des Katalysators wurden bei einer Temperatur von 150°C und einem Wasserstoffdruck von 250 bar 5 Gew.-Teile/h einer 50 %igen wäßrigen Lösung von Butin-2-diol-1,4 mit 2 500 Normvolumenteilen Wasserstoff hydriert. Zur Temperaturführung im Reaktor und zur Gewährleistung der erforderlichen Flüssigkeitsverteilung über das Katalysatorbett wurde der Butindiol-Zulauf mit 50 Volumenteilen/h Reaktoraustrag verdünnt. Die Hydrierwärme wurde über einen Wärmetauscher im Flüssigkeitskreislauf abgeführt. Nach Abtrennung der Gasphase von der Flüssigphase wurde das überschüssige Gas nach Ergänzung des verbrauchten Anteils durch Frischwasserstoff zum Reaktoreingang zurückgeführt.

Der Umsatz des Butin-2-diol-1,4 erfolgte nahezu vollständig. Nebenprodukte der Hydrierung (jeweils gerechnet wasserfrei) waren u.a.:
- Butanol: < 5 Gew.%
- 2-Methylbutandiol-1,4: < 0,2 Gew.%
- Buten-2-diol-1,4: < 0,1 Gew.%
- [2-(4-Hydroxi)-butoxi]-oxalen: < 0,3 Gew.%
- 4-Hydroxibutyraldehyd: < 0,5 Gew.%
- gamma-Butyrolacton: < 0,5 Gew.%

Zur weiteren Umsetzung der vier letztgenannten teilhydrierten Komponenten wurde der Austrag in einer zweiten Hydrierstufe im einfachen Durchgang am gleichen Katalysator bei Temperaturen von 180°C und einem Wasserstoffdruck von 250 bar nachhydriert.

Der anfallende Produktaustrag, der mindestens 94 Gew.% Butandiol-1,4 (berechnet wasserfrei) enthielt wurde destillativ zu Butandiol-1,4 rein aufgearbeitet. Auch nach einer Laufzeit von drei Monaten war noch kein deutlicher Druckverlust festzustellen. Weiterhin wurden keine Katalysatorbestandteile im Reaktoraustrag gefunden. Es wurden Butindiol-Durchsätze erzielt, welche um den Faktor drei höher lagen als beim folgenden Vergleichsversuch c).

### c) Vergleichsversuch

Die in Absatz b) beschriebene Hydrierung wurde unter Verwendung des im Beispiel der EP-B-18 569 beschriebenen Katalysators wiederholt. Schon nach einer Laufzeit von wenigen Wochen mußte die Katalysatorschüttung wegen Verstopfung ausgebaut werden.

### Beispiel 2

Die in Beispiel 1, Absatz b) beschriebene Hydrierung wurde über einen längeren Zeitraum fortgesetzt. Nach einer Betriebszeit von ca. 4 Monaten wurde eine Abnahme der Hydrierleistung des erfindungsgemäßen Katalysators beobachtet, welche hauptsächlich durch Ablagerungen anorganischer Bestandteile aus der eingesetzten technischen Butindiollösung verursacht wurde. Das Katalysatorbett wurde 24 Stunden mit 200°C heißem Kondensat gespült. Nach dieser Behandlung wies der Katalysator wieder annähernd seine ursprüngliche Aktivität auf. Das Spülwasser enthielt neben Spuren an Zirkoniumdioxid u.a. 0,1 Gew.% Silicium, 0,03 Gew.% Natrium, 0,01 Gew.% Kupfer sowie organische Bestandteile (< 5 Gew.%).

### Beispiel 3

### Hydrierung von 2-Ethylhexen-2-al

Ein kontinuierlich betriebener Hochdruckreaktor wurde mit dem nach Beispiel 1, Absatz a) hergestellten Katalysator gefüllt. Die Reduktion des oxidischen Katalysators erfolgte bei einer Temperatur von 270°C und einem Wasserstoffdruck von 150 bar.

An 1,5 Volumenteilen des reduzierten Katalysators wurden nun stündlich 3 Volumenteile 2-Ethylhexen-2-al (Gehalt: 95 bis 96 Gew.%) bei einer Temperatur von 140°C und einem Wasserstoffdruck von 250 bar hydriert. Der Frischzulauf wurde aus den in Beispiel 2 dargestellten Gründen mit 30 Teilen/h Reaktoraustrag verdünnt. Der durch die Hydrierung nicht verbrauchte Wasserstoff wurde durch Frischwasserstoff ergänzt und als Kreisgas zurückgeführt. Der Umsatz war nahezu quantitativ. Im Austrag wurden u.a. gefunden:
- 2-Ethylhexen-2-al max.: 0,01 Gew.%
- 2-Ethylhexanal: < 0,03 Gew.%
- 2-Ethylhexanol: > 95,9 Gew.%

Die Absenkung des Systemdrucks führte erwartungsgemäß zu einer Abnahme der Hydrierleistung. Bei 40 bar Betriebsdruck konnten unter Einhaltung der beschriebenen Austragsspezifikation noch 0,5 Volumenteile/h 2-Ethylhexen-2-al hydriert werden. Die Reaktorausträge enthielten in keinem Fall signifikante Mengen an gelösten oder ungelösten Katalysatorbestandteilen.

Die Durchsatzmengen, die pro Zeiteinheit erzielt wurden, liegen um den Faktor sechs höher als die Durchsätze, die man bei Einsatz der üblicherweise angewendeten Nickelkatalysatoren auf Siliciumdioxid erreicht.

Das aus dem Rohprodukt der Hydrierung durch Destillation hergestellte 2-Ethylhexanol zeichnet sich durch eine hohe Reinheit aus. Besonders hervorzuheben ist die geringe Schwefelsäurefarbzahl von 5 APHA, die nicht erreicht wird, wenn 2-Ethylhexen-2-al mit Hilfe des üblicherweise verwendeten Nickelkatalysators auf Siliciumdioxid zu 2-Ethylhexanol hydriert wird.

## Patentansprüche

1. Katalysator für die Hydrierung aliphatischer ungesättigter Verbindungen, der Nickel und Kupfer enthält, gekennzeichnet durch einen Gehalt von 20 bis 75 Gew.% Nickeloxid, 10 bis 75 Gew.% Zirkoniumdioxid und 5 bis 50 Gew.% Kupferoxid, jeweils bezogen auf den oxidischen, nicht reduzierten Katalysator.

2. Katalysator nach Anspruch 1, gekennzeichnet durch einen Gehalt an 30 bis 70 Gew.% Nickeloxid, 10 bis 60 Gew.% Zirkoniumdioxid und 5 bis 40 Gew.% Kupferoxid, jeweils bezogen auf den oxidischen, nicht reduzierten Katalysator.

3. Katalysator nach Anspruch 1, gekennzeichnet durch einen Gehalt an 40 bis 60 Gew.% Nickeloxid, 15 bis 50 Gew.% Zirkoniumdioxid und 10 bis 35 Gew.% Kupferoxid, jeweils bezogen auf den oxidischen, nicht reduzierten Katalysator.

4. Katalysator nach Anspruch 1, gekennzeichnet durch einen Gehalt an 35 bis 55 Gew.% Nickeloxid, 25 bis 45 Gew.% Zirkoniumdioxid, 10 bis 20 Gew.% Kupferoxid und 0,1 bis 5 Gew.% Molybdänoxid, jeweils bezogen auf den oxidischen, nicht reduzierten Katalysator.

5. Verfahren für die katalytische Hydrierung von aliphatischen ungesättigten Verbindungen mit einem wasserstoffhaltigen Gas im Temperaturbereich von 40 bis 200°C und bei einem Druck von 30 bis 320 bar, dadurch gekennzeichnet, daß man als Hydrierkatalysator die Katalysatoren gemäß den Ansprüchen 1 bis 4 verwendet.

6. Verfahren nach Anspruch 5, dadurch gekennzeichnet, daß man Butin-2-diol-1,4 zu Butandiol-1,4 hydriert.

7. Verfahren nach Anspruch 5, dadurch gekennzeichnet, daß man den Katalysator nach Abfall der Aktivität durch eine Behandlung mit Wasser bei Temperaturen von 100 bis 200°C und Drücken von 100 bis 320 bar reaktiviert.

8. Verfahren nach Anspruch 5, dadurch gekennzeichnet, daß man 2-Ethylhexen-2-al zu 2-Ethylhexanol hydriert.

## Claims

1. A catalyst for the hydrogenation of aliphatic, unsaturated compounds which contains nickel and copper, containing from 20 to 75 % by weight of nickel oxide, from 10 to 75 % by weight of zirconium dioxide and from 5 to 50 % by weight of copper oxide, in each case based on the oxidic, unreduced catalyst.

2. A catalyst as claimed in claim 1, containing from 30 to 70 % by weight of nickel oxide, from 10 to 60 % by weight of zirconium dioxide and from 5 to 40 % by weight of copper oxide, in each case based on the oxidic, unreduced catalyst.

3. A catalyst as claimed in claim 1, containing from 40 to 60 % by weight of nickel oxide, from 15 to 50 % by weight of zirconium dioxide and from 10 to 35 % by weight of copper oxide, in each case based on the oxidic, unreduced catalyst.

4. A catalyst as claimed in claim 1, containing from 35 to 55 % by weight of nickel oxide, from 25 to 45 % by weight of zirconium dioxide and from 10 to 20 % by weight of copper oxide and from 0.1 to 5 % by weight of molybdenum oxide, in each case based on the oxidic, unreduced catalyst.

5. A process for the catalytic hydrogenation of aliphatic, unsaturated compounds using a hydrogen-containing gas at from 40 to 200°C and at from 30 to 320 bar, which comprises using, as hydrogenation catalyst, a catalyst as claimed in any of claims 1 to 4.

6. A process as claimed in claim 5, wherein 2-butyne-1,4-diol is hydrogenated to butane-1,4-diol.

7. A process as claimed in claim 5, wherein the catalyst is reactivated, after a drop in activity, by treatment with water at from 100 to 200°C and at from 100 to 320 bar.

8. A process as claimed in claim 5, wherein 2-ethylhexen-2-al is hydrogenated to 2-ethylhexanol.

## Revendications

1. Catalyseur qui contient du nickel et du cuivre pour l'hydrogénation de composés aliphatiques insaturés, caractérisé par une teneur en oxyde de nickel de 20 à 75% en poids, en dioxyde de zirconium de 10 à 75% en poids et en oxyde de cuivre de 5 à 50% en poids, chaque fois par rapport au catalyseur à l'état oxydé non encore réduit.

2. Catalyseur selon la revendication 1, caractérisé par une teneur en oxyde de nickel de 30 à 70% en poids, en dioxyde de zirconium de 10 à 60% en poids et en oxyde de cuivre de 5 à 40% en poids, chaque fois par rapport au catalyseur à l'état oxydé non encore réduit.

3. Catalyseur selon la revendication 1, caractérisé par une teneur en oxyde de nickel de 40 à 60% en poids, en dioxyde de zirconium de 15 à 50% en poids et en oxyde de cuivre de 10 à 35% en poids, chaque fois par rapport au catalyseur à l'état oxydé non encore réduit.

4. Catalyseur selon la revendication 1, caractérisé par une teneur en oxyde de nickel de 35 à 55% en poids, en dioxyde de zirconium de 25 à 45% en poids, en oxyde de cuivre de 10 à 20% en poids et en oxyde de molybdène de 0,1 à 5% en poids, chaque fois par rapport au catalyseur à l'état oxydé non encore réduit.

5. Procédé d'hydrogénation catalytique de composés aliphatiques insaturés avec un gaz contenant de l'hydrogène, dans la gamme de température de 40 à 200°C et sous une pression de 30 à 320 bar, caractérisé en ce qu'on utilise, comme catalyseur d'hydrogénation, les catalyseurs selon l'une quelconque des revendications 1 à 4.

6. Procédé selon la revendication 5, caractérisé en ce qu'on transforme du butyne-2-diol-1,4 en butanediol-1,4 par hydrogénation.

7. Procédé selon la revendication 5, caractérisé en ce qu'à la suite de la baisse d'activité du catalyseur, on réactive celui-ci par un traitement par de l'eau à des températures de 100 à 200°C et sous des pressions de 100 à 320 bar.

8. Procédé selon la revendication 5, caractérisé en ce qu'on transforme du 2-éthylhexène-2-al en 2-éthylhexanol par hydrogénation.
